# EUROPEAN PATENT APPLICATION

(11) **EP 2 343 283 A1**
(43) Date of publication of application: **13.07.2011**
(21) Application number: 09820394.6
(22) Date of filing: 06.10.2009
(51) Int. Cl.: C07D 239/52, A01N 43/54, A01P 13/02

(54) **OPTICALLY ACTIVE DIFLUOROMETHANESULFONANILIDE DERIVATIVE AND HERBICIDE**

(30) Priority: 17.10.2008 JP 2008268311
(71) Applicant: Kumiai Chemical Industry CO., LTD., Tokyo 110-0008 (JP); IHARA CHEMICAL INDUSTRY CO., LTD., Taito-ku Tokyo 110-0008 (JP)
(72) Inventor: INOUE, Jun, Iwata-shi Shizuoka 437-1213 (JP); KAWASAKI, Hiroshi, Tokyo 110-0008 (JP); ASAKURA, Sohei, Tokyo 110-0008 (JP)
(74) Representative: Vossius & Partner
(86) International application number: PCT/JP2009/005174
(87) International publication number: WO 2010/044215

(57) **Abstract**

The present invention aims at providing an optically active difluoromethanesulfonanilide derivative exhibiting an excellent herbicidal activity at a low dose.

An optically active (R)-2'-(4,6-dimethoxy-pyrimidin-2-yl)hydroxymethyl-1,1-difluoromethanesulfonanilide derivative is represented by the formula (I) 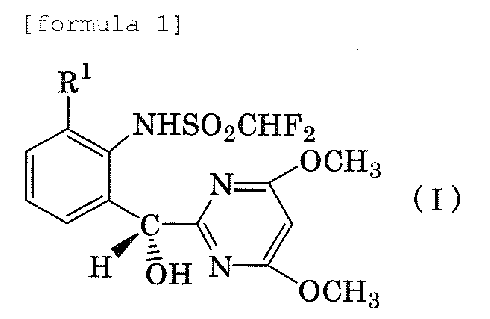 (in the formula, R¹ is a hydrogen atom, a C1-C4 alkyl group or a C1-C4 alkoxy C1-C4 alkyl group).
and this difluoromethanesulfonanilide derivative is produced by subjecting to optical resolution a racemate of 2'-(4,6-dimethoxypyrimidin-2-yl)hydroxymethyl-1,1-difluoromethanesulfonanilide derivative.

## Description

### TECHNICAL FIELD

The present invention relates to an optically active (R)-2'-(4,6-dimethoxypyrimidin-2-yl)hydroxymethyl-1,1-difluoromethanesulfonanilide derivative and a herbicide containing the derivative as an active ingredient.

### BACKGROUND ART

As the properties to be possessed by the herbicide used in cultivation of important crops, particularly paddy rice, there have been mentioned excellent herbicidal activity and wide weeding spectrum at a low dose, sufficient safety to rice, etc. In recent years, a herbicide having an excellent herbicidal activity at a low dose has been required particularly from an environmental standpoint. Meanwhile, Patent Document 1 discloses a 2'-(4,6-dimethoxypyrimidin-2-yl)hydroxymethyl-1,1-difluoromethanesulfonanilide derivative and describes that the compound is useful as a herbicide. The compound of the Patent Document 1 has an asymmetric carbon atom and suggests the presence of optical isomers. However, the Patent Document 1 makes no mention on the optical isomers.

### PRIOR ART DOCUMENT

### Patent Document

Patent Document 1: JP-A-2000-44546

### SUMMARY OF THE INVENTION

### Task to Be Achieved by the Invention

The task of the present Invention has been made in view of the above situation. The present invention aims to provide an optically active (R) -2' - ( 4,6-dimethoxypyrimidin-2-yl)hydroxymethyl-1,1-difluoromethanesulfonanilide derivative exhibiting an excellent herbicidal activity at a low dose and a herbicide containing the derivative as an active ingredient.

### Means for Achieving the Task

The present inventors made an intensive study. As a result, it was found that, in the compound described in the Patent Document 1, a particular optical isomer, i.e. a (R)-2'-(4,6-dimethoxypyrimidin-2-yl)hydroxymethyl-1,1-difluoromethanesulfonanilide derivative, as compared with corresponding another optical isomer and corresponding racemate, exhibits far superior herbicidal activity. The finding has led to the completion of the present invention.

The present invention relates to the following inventions.

(1) An optically active (R)-2'-(4,6-dimethoxypyrimidin-2-yl)hydroxymethyl-1,1-difluoromethanesul-fonanilide derivative represented by the formula (I)

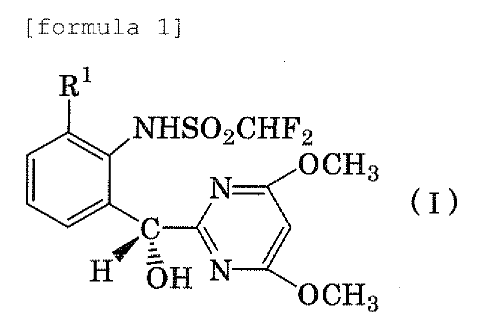

(in the formula, R¹ is a hydrogen atom, a C1-C4 alkyl group or a C1-C4 alkoxy C1-C4 alkyl group).

(2) An optically active (R) -2' - ( 4,6 - dimethoxypyrimidin-2-yl)hydroxymethyl-1,1-difluoromethanesulfonanilide derivative according to (1), wherein R¹ is a methoxymethyl group.

(3) A herbicide characterized by containing, as an active ingredient, an optically active (R)-2'-(4,6-dimethoxypyrimidin-2-yl)hydroxymethyl-1,1-difluoromethanesulfonanilide derivative according to (1) or (2).

(4) A method for producing an optically active (R)-2'-(4,6-dimethoxypyrimidin-2-yl)hydroxymethyl-1,1-difluoromethanesulfonanilide derivative, characterized by subjecting to optical resolution a racemate of 2'-(4,6-dimethoxypyrimidin-2-yl)hydroxymethyl-1,1-difluoromethanesulfonanilide derivative.

### Effects of the Invention

The optical isomer of the present invention, i.e. an (R)-2'-(4,6-dimethoxypyrimidin-2-yl)hydroxymethyl-1,1-difluoromethanesulfonanilide derivative, as compared with corresponding other optical isomer, i.e. an (S)-2'-(4,6-dimethoxypyrimidin-2-yl)hydroxymethyl-1,1-difluoromethanesulfonanilide derivative and corresponding racemate, i.e. an (RS)-2'-(4,6-dimethoxypyrimidin-2-yl)hydroxymethyl-1,1-difluoromethanesulfonanilide derivative, exhibits, at a low dose, a herbicidal activity to a wide range of weeds over a long application period and has an excellent control effect particularly to weeds of paddy field. Therefore, the present optical isomer is useful as a herbicide.

### MODE FOR CARRYING OUT THE INVENTION

The present invention is described in detail below.

In the formula (I), the substituent group R¹ is a hydrogen atom, a C1-C4 alkyl group or a C1-C4 alkoxy C1-C4 alkyl group. The C1-C4 alkyl group is a straight chain or branched chain alkyl group having 1 to 4 carbon atoms unless otherwise specified, and there can be mentioned, for example, groups such as methyl, ethyl, propyl, isopropyl, butyl, isobutyl, sec-butyl and the like.

The C1-C4 alkoxy C1-C4 alkyl group is a straight chain or branched chain alkyl group having 1 to 4 carbon atoms, substituted by a straight chain or branched chain alkoxy group having 1 to 4 carbon atoms, unless otherwise specified, and there can be mentioned, for example, methoxymethyl group, ethoxymethyl group, propoxymethyl group, isopropoxymethyl group, butoxymethyl group, isobutoxymethyl group, sec-butoxymethyl group, methoxyethyl group, ethoxyethyl group, propoxyethyl group, isopropoxyethyl group, butoxyethyl group, isobutoxyethyl group, sec-butoxyethyl group, methoxypropyl group, ethoxypropyl group, propoxypropyl group, isopropoxypropyl group, butoxypropyl group, isobutoxypropyl group, sec-butoxypropyl group, methoxybutyl group, ethoxybutyl group, propoxybutyl group, isopropoxybutyl group, butoxybutyl group, isobutoxybutyl group, sec-butoxybutyl group and 2-methoxypropyl group.

The expression "(R)-2'-(4,6-dimethoxypyrimidin-2-yl)hydroxymethyl-1,1-difluoromethanesulfonanilide derivative" generally means that the compound is an (R) isomer. It is preferred that the same is true also in the derivative of the invention of the present application; however, the (R)-2'-(4,6-dimethoxypyrimidin-2-yl)hydroxymethyl-1,1-difluoromethanesulfonanilide derivative of the invention of the present application may contain a small amount of an (S) isomer, because the (R) isomer may change into an (S) isomer with the elapse of time owing to various conditions (e.g. light) of natural environment.

The (R)-2'-(4,6-dimethoxypyrimidin-2-yl)hydroxylmethyl-1,1-difluoromethanesulfonanilide derivative of the present invention can be produced by the production method of the present invention of subjecting to optical resolution a racemate represented by the formula (II)

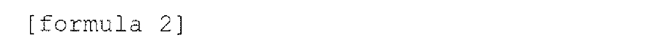

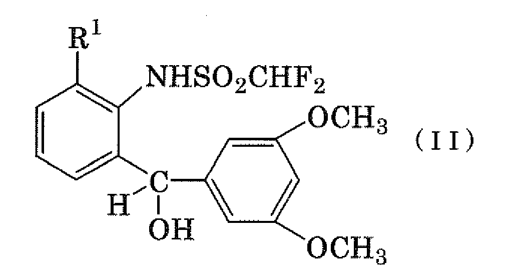

(in the formula, R¹ is a hydrogen atom, a C1-C4 alkyl group or a C1-C4 alkoxy C1-C4 alkyl group).

For the optical resolution, there can be employed, for example, a method of resolving a racemate into an (R) optical isomer and an (S) optical isomer using a column for high-performance liquid chromatography used for separation of optical isomers. The column for high-performance liquid chromatography used for separation of optical isomers is available commercially. There can be mentioned, for example, CHIRAL PAK AD produced and marketed by Daicel Chemical Industries, Ltd., in which 1,4-poly-2,3,6-tri-o-phenylcarbamoyl-β-D-glucoside loaded on a silica gel is filled as a resolving agent.

As the solvent used for the optical resolution, there can be mentioned, for example, aliphatic hydrocarbons such as hexane, heptane and the like; alcohols such as methanol, ethanol, propanol, 2-propanol, butanol and the like; halogenated hydrocarbons such as dichloromethane, chloroform and the like; ethers such as diethyl ether, 1,2-dimethoxyethane, diisopropyl ether, tetrahydrofuran, dioxane and the like; nitriles such as acetonitrile and the like; acetic acid; water; and mixed solvent thereof.

A racemate (RS)-2'-(4,6-dimethoxypyrimidin-2-yl)hydroxymethyl-1,1-difluoromethanesulfonanilide derivative can be produced by the method described in the above-mentioned Patent Document 1.

The temperature and time of the optical resolution can each be varied over a wide range. Generally, the temperature is -20 to 60°C, preferably 5 to 50°C. The time is 0.01 hour to 50 hours, preferably 0.1 to 2 hours.

The herbicide of the present invention contains, as an active ingredient, the thus-produced optically active (R)-2'-(4,6-dimethoxypyrimidin-2-yl)hydroxymethyl-1,1-difluoromethanesulfonanilide derivative.

The herbicide of the present invention may contain, as necessary, additive ingredients (carriers) ordinarily employed in agricultural chemical formulations.

As the addictive ingredients, there can be mentioned carriers such as solid carrier and liquid carrier, surfactant, binder, tackifier, thickener, coloring agent, spreader, sticker, antifreezing agent, anticaking agent, collapsing agent, decomposition inhibitor and the like. If necessary, an anticeptic agent, a piece of plant and the like may also be employed as additive ingredients.

These additive ingredients may be used singly or in combination of two or more kinds.

Description is made on the above additive ingredients.

As the solid carrier, there can be mentioned, for example, natural minerals such as quartz, clay, kaolinite, pyrophyllite, sericite, talc, bentonite, Japanese acid clay, attapulgite, zeolite, diatomaceous earth and the like; inorganic salts such as calcium carbonate, ammonium sulfate, sodium sulfate, potassium chloride and the like; organic solid carriers such as synthetic silicic acid, synthetic silicate, starch, cellulose, plant powder and the like; and plastic carriers such as polyethylene, polypropylene, polyvinylidene chloride and the like.

As the liquid carrier, there can be mentioned, for example, alcohols, i.e. monohydric alcohols such as methanol, ethanol, propanol, isopropanol, butanol and the like and polyhydric alcohols such as ethylene glycol, diethylene glycol, propylene glycol, hexylene glycol, polyethylene glycol, polypropylene glycol, glycerine and the like; polyhydric alcohol derivatives such as propylene glycol ether and the like; ketones such as acetone, methyl ethyl ketone, methyl isobutyl ketone, diisobutyl ketone, cyclohexanone, isophorone and the like; ethers such as ethyl ether, dioxane, cellosolve, dipropyl ether, tetrahydrofuran and the like; aliphatic hydrocarbons such as normal paraffin, naphthene, isoparaffin, kerosene, mineral oil and the like; aromatic hydrocarbons such as benzene, toluene, xylene, solvent naphtha, alkylnaphthalene and the like; halogenated hydrocarbons such as dichloroethane, chloroform, carbon tetrachloride and the like; esters such as ethyl acetate, diisopropyl phthalate, dibutyl phthalate, dioctyl phthalate, dimethyl adipate and the like; lactones such as γ-butyrolactone and the like; amides such as dimethylformamide, diethylformamide, dimethylacetamide, N-alkylpyrrolidinone and the like; nitriles such as acetonitrile and the like; sulfur compounds such as dimethyl sulfoxide and the like; vegetable oils such as soybean oil, rapeseed oil, cottonseed oil, castor oil and the like; and water.

As to the surfactant, there is no particular restriction; however, the surfactant is preferred to become a gel or exhibiting swelling property in water. Examples thereof may include nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkylether, polyoxyethylene alkylphenylether, polyoxyethylene dialkylphenylether, polyoxyethylene alkylphenylether formaldehyde condensate, polyoxyethylene polyoxypropylene block polymer, alkylpolyoxyethylene polypropylene block polymer ether, polyoxyethylene alkylamine, polyoxyethylene fatty acid amide, polyoxyethylene fatty acid bisphenylether, polyalkylene benzyl-phenyl ether, polyoxyalkylene styrylphenyl ether, acetylene diol, polyoxyalkylene-added acetylene diol, polyoxyethylene ether silicone, ester silicone, fluorochemical surfactant, polyoxyethylene castor oil and polyoxyethylene hydrogenated castor oil; anionic surfactants such as alkyl sulfate, polyoxyethylene alkylether sulfate, polyoxyethylene alkylphenylether sulfate, polyoxyethylene styrylphenyl ether sulfate, alkyl benzene sulfonate, lignin sulfonate, alkyl sulfosuccinate, naphthalene sulfonate, alkyl naphthalene sulfonate, naphthalenesulfonic acid formaldehyde condensate salt, alkylnaphthalenesulfonic acid formaldehyde condensate salt, fatty acid salt, polycarboxylate, N-methyl-fatty acid sarcosinate, resinate, polyoxyethylene alkylether phosphate and polyoxyethylene alkylphenylether phosphate; cationic surfactants such as laurylamine hydrochloride, stearylamine hydrochloride, oleylamine hydrochloride, stearylamine acetate, stearylaminopropylamine acetate, alkyltrimethylammonium chloride and alkyldimethylbenzalkonium chloride; amino acid or betaine type amphoteric surfactants; and the like.

As the binder and the tackifier, there can be mentioned, for example, carboxymethyl cellulose or its salt, dextrin, water-soluble starch, xanthan gum, guar gum, sucrose, polyvinylpyrrolidone, gum arabic, polyvinyl alcohol, polyvinyl acetate, sodium polyacrylate, polyethylene glycol having an average molecular weight of 6,000 to 20,000, polyethylene oxide having an average molecular weight of 100,000 to 5,000,000, and natural phospholipid (e.g. cephalinic acid or lecithin).

As the thickener, there can be mentioned, for example, water-soluble high-molecular compounds such as xanthan gum, guar gum, carboxymethyl cellulose, polyvinyl pyrrolidone, carboxyvinyl polymer, acrylic polymer, starch derivative, polysaccharide and the like; and inorganic fine powders such as high-purity bentonite, white carbon and the like.

As the coloring agent, there can be mentioned, for example, inorganic pigments such as iron oxide, titanium oxide and Prussian blue; and organic dyes such as alizarine dye, azo dye and metal phthalocyanine dye.

As the spreader, there can be mentioned, for example, silicone type surfactant, cellulose powder, dextrin, processed starch, polyaminocarboxylic acid chelate compound, crosslinked polyvinylpyrrolidone, methacrylic acid copolymer, half-ester of polyhydric alcohol polymer and dicarboxylic acid anhydride, and water-soluble salt of polystyrenesulfonic acid.

As the sticker, there can be mentioned, for example, surfactants such as sodium dialkylsulfosuccinic acid, polyoxyethylene alkyl ether, polyoxyethylene alkylphenyl ether, polyoxyethylene fatty acid ester and the like; paraffin; terpene; polyamide resin; polyacrylic acid salt; polyoxyethylene; wax; polyvinyl alkyl ether; alkylphenol formalin condensate; and synthetic resin emulsion.

As the antifreezing agent, there can be mentioned, for example, polyhydric alcohols such as ethylene glycol, diethylene glycol, propylene glycol, glycerine and the like.

As the anticaking agent, there can be mentioned, for example, polysaccharides such as starch, alginic acid, mannose, galactose and the like; polyvinylpyrrolidone; white carbon; ester gum; and petroleum resin.

As the collapsing agent, there can be mentioned, for example, sodium tripolyphosphate, sodium hexametaphosphate, stearic acid metal salt, cellulose powder, dextrin, copolymer of methacrylic acid ester, polyvinylpyrrolidone, polyaminocarboxylic acid chelate compound, sulfonated styrene-isobutylene-maleic anhydride copolymer, and starch-polyacrylonitrile graft copolymer.

As the decomposition inhibitor, there can be mentioned, for example, drying agents such as zeolite, quick lime, magnesium oxide and the like; anti-oxidants such as phenol type, amine type, sulfur type, phosphoric acid type and the like; and ultraviolet-absorbers such as salicylic acid type, benzophenone type and the like.

As the antiseptic agent, there can be mentioned, for example, potassium sorbate and 1,2-benzthiazolin-3-one.

As the piece of plant, there can be mentioned, for example, sawdust, coconut shell, corn rachis and tobacco stem.

When the herbicide of the present invention contains the above-mentioned additive ingredients, the content thereof in terms of mass % is ordinarily 5 to 95%, preferably 20 to 90% in the case of carrier, ordinarily 0.1% to 30%, preferably 0.5 to 10% in the case of surfactant, and 0.1 to 30%, preferably 0.5 to 10% in the case of other additives.

The herbicide of the present invention is made into a desired formulation such as liquid formulation, emulsifiable concentrate, wettable powder, dust, oil solution, water dispersible granule, flowable, granule, Jumbo formulation, suspoemulsion, MAMETSUBU (registered trade name) agent or the like and is used.

The present herbicide can be mixed, in making into a desired formulation, with at least one kind of agricultural chemical (selected from other herbicide, insecticide, germicide, plant growth-controlling agent, etc.), safener, fertilizer, etc., or can be used together with them.

In actual use, the present herbicide is diluted into an appropriate concentration and sprayed, or is applied per se.

Examples of the known herbicide compound or plant growth-controlling agent which may be mixed or used together with the present herbicide, are shown below.

2,3,6-TBA, 2,4-D, 2,4-DB, DNOC, EPTC, ethoxyfen-ethyl, MCPA, MCPA-thioethyl, MCPB, S-metolachlor, TCA, ioxynil, aclonifen, azafenidin, acifluorfen, azimsulfuron, asulam, acetochlor, atrazine, anilofos, amicarbazone, amido-sulfuron, amitrole, aminopyralid, amiprophos-methyl, ametryn, alachlor, alloxydim, ancymidol, iodosulfulon-methyl-sodium, isouron, isoxachlortole, isoxaflutole, isoxaben, isoproturon, imazaquin, imazapyr, imazamethabenz-methyl, imazapic, imazamox, imazethapyr, imazosulfuron, indanofan, esprocarb, ethametsulfuron-methyl, ethalfluralin, ethidimuron, ethoxy-sulfuron, ethofumesate, etobenzanid, oxadiazon, oxadiargyl, oxaziclomefone, oxasulfuron, oxyfluorfen, oryzalin, orbencarb, cafenstrole, carfentrazone-ethyl, karbutilate, carbetamide, quizalofop, quizalofop-P-ethyl, quizalofop-P-tefuryl, quizalofop-ethyl, quinclorac, quinmerac, cumyluron, glyphosate, glyphosate-trimesium, glufosinate-ammonium, glufosinate-sodium, clethodim, clodinafop-propargyl, clopyralid, clomazone, chlomethoxyfen, clomeprop, cloransuiam-methyl, chloramben, chloridazon, chlorimuron-ethyl, chlorsulfuron, chlorthal-dimethyl, chlorthiamid, chlorpropham, chlormequat chloride, chloroxuron, chlorotoluron, chlorobromuron, cyanazine, diuron, dicamba, cycloate, cycloxydim, diclosulam, cyclosulfamuron, dichlobenil, diclofop-methyl, dichlorprop, dichlorprop-P, diquat dibromide, dithiopyr, siduron, dinitramine, cinidon-ethyl, cinosulfuron, dinoseb, dinoterb, cyhalofop-butyl, diphenamid, difenzoquat, diflufenican, diflufenzopyr, diflumetorim, simazine, dimethachlor, dimethametryn, dimethenamid, simetryn, dimepiperate, dimefuron, cinmethylin, sulcotrione, sulfentrazone, sulfosulfuron, sulfometuronmethyl, sethoxydim, terbacil, daimuron, dalapon, thiazopyr, tiocarbazil, thiobencarb, thidiazimin, thidiazuron, thifensulfuron-methyl, desmedipham, desmetryne, thenylchlor, tebutam, tebuthiuron, tepraloxydim, tefuryltrion, terbuthylazine, terbutryn, terbumeton, tembotrione, topramezone, tralkoxydim, triaziflam, triasulfuron, triallate, trietazine, triclopyr, triflusulfuron-methyl, tritosulfuron, trifluralin, trifloxysulfuron-sodium, tribenuron-methyl, naptalam, naproanilide, napropamide, nicosulfuron, neburon, norflurazon, vernolate, paraquat dichloride, haloxyfop, haloxyfop-P-methyl, halosulfuron-methyl, pinoxaden, picloram, picolinafen, bispyribacsodium, bifenox, piperophos, pyraclonil, pyrasulfotole, pyrazoxyfen, pyrazosulfuron-ethyl, pyrazolynate, bilanafos, pyraflufen-ethyl, pyridafol, pyrithiobac-sodium, pyridate, pyriftalid, pyributicarb, pyribenzoxim, primisulfuron-methyl, pyriminobac-methyl, pyroxsulam, fenuron, fenaxaprop-P-ethyl, fenoxaprop-ethyl, fenclorim, fentrazamide, phenmedipham, foramsulfuron, butachlor, butafenacil, butamifos, butylate, butralin, butroxydim, flazasulfuron, flamprop-M, fluazifop-butyl, fluazifop-P-butyl, fluazolate, fluometuron, fluoroglycofen-ethyl, flucarbazone-sodium, flucetosulfuron, fluthiacet-methyl, flupyrsulfuron-methyl-sodium, flufenacet, flufenpyr-ethyl, flupropanate, flupoxame, flumioxazin, flumiclorac-pentyl, flumetsulam, fluridone, flurtamone, flurprimidol, fluroxypyr, flurochloridone, pretilachlor, prodiamine, prosulfuron, prosulfocarb, propaquizafop, propachlor, propazine, propanil, propyzamide, propisochlor, propham, profluazol, propoxycarbazone, propoxycarbazone-sodium, profoxydim, bromacil, prometryn, prometon, bromoxynil, bromofenoxim, bromobutide, florasulam, hexazinone, pethoxamid, benazolin, penoxsulam, beflubutamid, pebulate, bencarbazone, pendimethalin, benzfendizone, bensulide, bensulfuron-methyl, benzobicyclon, benzofenap, bentazone, pentanochlor, pentoxazone, benfluralin, benfuresate, fosamine, fomesafen, forchlorfenuron, maleic hydrazide, mecoprop, mecoprop-P, mesosulfuron-methyl, mesotrione, metazachlor, methabenzthiazuron, metamitron, metamifop, methyl daimuron, metoxuron, metosulam, metsulfuronmethyl, metobromuron, metobenzuron, metolachlor, metribuzin, mepiquat chloride, mefenacet, monolinuron, molinate, lactofen, linuron, rimsulfuron, lenacil, prohexadione-calcium, trinexapac-ethyl, pyroxasulfone, fenoxasulfone and an isoxazoline derivative represented by the formula [C]

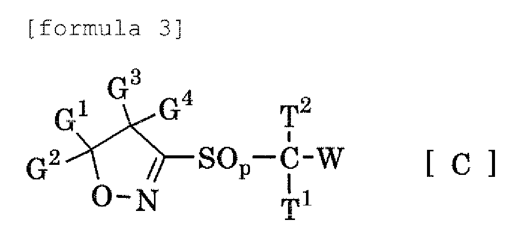

[in the formula, p is an integer of 0 to 2;
T¹ and R² are each independently a hydrogen atom, a halogen atom, a cyano group, a lower alkoxycarbonyl group or a C₁ to C₆ alkyl group;
G¹, G², G³ and G⁴ are each independently a hydrogen atom, a C₁ to C₆ alkyl group or a C₁ to C₆ haloalkyl group;
*W* is a phenyl group (substituted by 1 to 5 same or different V's) or a pyrazolyl group (substituted by 1 to 4 same or different V's):
V is a hydrogen atom, a C₁ to C₆ alkyl group {which may be substituted by 1 to 3 same or different halogen atoms, a C₁ to C₆ alkoxy group, a hydroxyl group, a C₁ to C₆ alkylthio group, a C₁ to C₆ alkylsulfinyl group, a C₁ to C₆ alkylsulfonyl group, a C₁ to C₆ alkylamino group, a C₁ to C₆ dialkylamino group, a cyano group, or phenoxy (which may be substituted)}, a C₁ to C₆ alkoxy group (which may be substituted by 1 to 3 same or different halogen atoms, a C₁ to C₆ alkoxy group, a C₂ to C₆ alkenyl group, a C₂ to C₆ alkinyl group, a C₁ to C₆ alkoxycarbonyl group, a C₁ to C₆ alkylcarbonyl group or a C₃ to C₈ cycloalkyl group), a C₃ to C₈ cycloalkyloxy group, or a halogen atom].

Examples of the known phytotoxicity-reducing compound which may be mixed or used together with the present herbicide, are shown below.

benoxacor, furilazole, dichlormid, dicyclonone, DKA-24(N¹,N²-diallyl-N²-dichloroacetylglycineamide) , AD-67 (4-dichloroacetyl-1-oxa-4-azaspiro-[4.5]decane), PPG-1292 (2,2-dichloro-N-(1,3-dioxane-2-ylmethyl)-N-(2-propenyl)acetamide), R-29148(3-dichloroacetyl-2,2,5-trimethyl-1,3-oxazolidine), cloquintcet-mexyl, 1,8-naphthalic Anhydride, mefenpyr-diethyl, mefenpyr, fenchlorazole-ethyl, fenclorim, MG-191 (2-dichloromethyl-2-methyl-1,3-dioxane), cyometrinil, flurazole, fluxofenim, isoxandifen-ethyl, mecoprop, MCPA, daimuron, 2,4-D, isoxadifen, MON4660, oxabetrinil or cyprosulfamide etc.

Examples of the known germicide compound which may be mixed or used together with the present herbicide, are shown below.

acibenzolar-S-methyl, azoxystrobin, ametocradin, amisulbrom, aldimorph, isotianil, isopyrazam, isoprothiolane, ipconazole, iprodione, iprovalicarb, iprobenfos, imazalil, iminoctadine-albesilate, iminoctadine-triacetate, imibenconazole, edifenphos, ethaboxam, ethoxyquin, etridiazole, enestroburin, epoxiconazole, oxadixyl, oxazinylazole, oxycarboxin, oxytetracycline, oxpoconazole fumarate, oxolinic acid, octhilinone, ofurace, orysastrobin, o-phenylphenol, kasugamycin, captafol, carpropamid, carbendazim, carboxin, quinoxyfen, chinomethionat, captan, quintozene, guazatine, kresoximmethyl, chlorothalonil (TPN), chloroneb, cyazofamid, diethofencarb, diclocymet, dichlofluanid, diclomezine, dicloran, dithianon, diniconazole, zineb, dinocap, biphenyl, diphenylamine, difenoconazole, difenzoquat methyl sulfate, cyflufenamid, diflumetorim, cyproconazole, cyprodinil, simeconazole, dimethomorph, cymoxanil, dimoxystrobin, ziram, silthiofam, streptomycin, spiroxamine, zoxamide, dazomet, tiadinil, thiabendazole, thiophanate-methyl, thifluzamide, thiram, tecnazene, tecloftalam, tetraconazole, debacarb, tebuconazole, tebufloquin, dodine, dodemorph, triadimenol, triadimefon, triazoxide, tricyclazole, triticonazole, tridemorph, triflumizole, trifloxystrobin, triforine, tolylfluanid, tolclofos-methyl, tolnifanide, nabam, nitrothal-isopropyl, nuarimol, validamycin, valifenalate, bixafen, picoxystrobin, bitertanol, piperalin, hymexazol, pyraclostrobin, pyrazophos, pyrifenox, pyributicarb, pyribencarb, pyrimethanil, pyrametostrobin, pyraoxystrobin, pyroquilon, vinclozolin, ferbam, famoxadone, fenamidone, fenarimol, fenoxanil, ferimzone, fenbuconazole, fenfuram, fenpropidin, fenpropimorph, fenhexamid, folpet, phthalide, bupirimate, fuberidazole, furametpyr, furalaxyl, fluazinam, fluoxastrobin, fluopicolide, fluopyram, fluoroimide, fluquinconazole, fludioxonil, flusilazole, flusulfamide, flutolanil, flutriafol, flumorph, proquinazid, prochloraz, procymidone, prothioconazole, bronopol, propamocarb-hydrochloride, propiconazole, propineb, probenazole, bromuconazole, hexaconazole, benalaxyl, benalaxyl-M, benomyl, pefurazoate, penconazole, pencycuron, benthiavalicarbisopropyl, penthiopyrad, boscalid, fosetyl-alminium, polyoxin, polycarbamate, mancozeb, mandipropamid, maneb, myclobutanil, mildiomycin, methasulfocarb, metam, metalaxyl, metalaxyl-M, metconazole, metominostrobin, metrafenone, mepanipyrim, mepronil, copper sulfate, copper hydroxide, cuprous oxide, copper oxychloride, oxyquinoline sufate, copper (nonylphenyl) sulphonate, oxine-copper, mancopper, silver, sulfur, potassium bicarbonate, (Bordeaux mixture) etc.

Examples of the known insecticide, miticide or nematicide compound which may be used mixed or used together with the present herbicide, are shown below.

1,3-dichloropropene, DCIP, DNOC, EPN, acrinathrin, azamethiphos, azinphos-ethyl, azinphos-methyl) , acequinocyl, acetamiprid, acetoprol, acephate, azocyclotin, abamectin, amitraz, alanycarb, aldicarb, alpha-cypermethrin, allethrin, isoxathion, isofenphos-methyl, isocarbophos, isoprocarb, imicyafos, imidacloprid) , imiprothrin, indoxacarb, esfenvalerate, ethiofencarb, ethion, ethiprole, etoxazole, etofenprox, ethoprophos, emamectin, endosulfan, empenthrin, oxamyl, oxydemeton-methyl, omethoate, cadusafos, karanjin, cartap, carbaryl, carbosulfan, carbofuran, gamma-cyhalothrin, xylylcarb, quinalphos, kinoprene, chinomethionat, coumaphos, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, cyazypyr, cyanophos, diafenthiuron, dienochlor, cyenopyrafen, dicrotophos, dichlofenthion, cycloprothrin, dichlorvos, dicofol, dicyclanil, disulfoton, dinotefuran, dinobuton, cyhalothrin, cyphenothrin, cyfluthrin, diflubenzuron, cyflumetofen, diflovidazin, cyhexatin, cypermethrin, dimethylvinphos, dimethoate, silafluofen, cyromazine, spinetoram, spinosad, spirodiclofen, spirotetramat, spiromesifen, sulcofuron-sodium, sulfluramid, sulfotep, sulfoxaflor, zeta-cypermethrin, diazinon, tau-fluvalinate, thiacloprid, thiamethoxam, thiodicarb, thiocyclam, thiosultap, thiofanox, thiometon, tetrachlorvinphos, tetradifon, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos) , trichlorfon, triflumuron, trimethacarb, tolfenpyrad, naled, nicotine, nitenpyram, novalaron, noviflumuron, hydroprene, vamidothion, parathion, parathion-methyl, halfenprox, halofenozide, bioallethrin, bioresmethrin, bistrifluron, hydramethylnon, bifenazate, bifenthrin, pymetrozine, pyraclofos, pyridaphenthion, pyridaben, pyridalyl, pyrifluquinazon, pyriproxyfen, pirimicarb, pyrimidifen, pirimiphos-methyl, famphur, fipronil, fenazaquin, fenamiphos) , fenitrothion, fenoxycarb, fenothiocarb, phenothrin, fenobucarb, fenthion, phenthoate, fenvalerate, fenpyroximate, fenbutatin oxide, fenpropathrin, butocarboxim, butoxycarboxim, buprofezin, furathiocarb, prallethrin, fluacrypyrim, flucycloxuron, flucythrinate, flusulfamide, fluvalinate, flupyrazofos, flufenerim, flufenoxuron, flubendiamide, flumethrin, flurimfen, prothiofos, flonicamid, propaphos, propargite, profenofos, propetamphos, propoxur, bromopropylate, beta-cyfluthrin, hexythiazox, hexaflumuron, heptenophos, permethrin, bensultap, benzoximate, bendiocarb, benfuracarb, phoxim, phosalone, fosthiazate, phosphamidon, phosmet, formetanate, phorate, malathion, milbemectin, mecarbam, mesulfenfos, methomyl, metaflumizon, methamidophos, metham, methiocarb, methidathion, methyl isothiocyanate, methoxychlor, methoxyfenozide, methothrin, metofluthrin, methoprene, mevinphos, monocrotophos, lambda-cyhalothrin, lufenuron, resmethrin, lepimectin, rotenone) , Bacillus thuringiensis etc.

The content of the active ingredient in the present herbicide can be determined appropriately so as to meet the requirement. However, it is 0.01 to 10% (mass), preferably 0.05 to 5% (mass) when the present herbicide is made into a powder, a granule, etc. When the present herbicide is made into an emulsifiable concentrate, a wettable powder, etc., the content of the active ingredient is determined appropriately in a range of 1 to 50% (mass), preferably 5 to 30% (mass). When the present herbicide is made into a flowable, etc., the content of the active ingredient is determined appropriately in a range of 1 to 40% (mass), preferably 5 to 30% (mass).

The application amount of the present herbicide varies depending upon the weed of target, the tendency of weed emergence, the conditions of environment, the use form of the herbicide, etc. However, when the present herbicide is used per se as in the cases of powder, granule, etc., the active ingredient is appropriately selected in a range of 1 g to 50 kg, preferably 10 g to 10 kg per 1 hectare. When the present herbicide is used in a liquid state as in the cases of emulsion, wettable powder, flowable, etc., the active ingredient is appropriately selected in a range of 0.1 to 50,000 ppm, preferably 10 to 10, 000 ppm.

The herbicide of the present invention can be used in upland field, paddy field, orchard, etc. by foliage application, soil application, water surface application and other applications. The present herbicide can be used also for killing of general weeds in fallow fields, ridges between rice fields, farm roads, waterways, grass farms, graveyards, parks, roads, playgrounds, vacant lots around buildings, lands under cultivation, neighborhood of railways, forests, etc.

The herbicide of the present invention exhibits an excellent herbicidal effect, over a long period from before germination to growth period, to various weeds which cause problems in upland field and orchard, such as smart-weeds such as Polygonum lapathifolium L., Polygonum longisetum De Bruyn and Rumex japonicus Houtt.; amaranthus such as Amaranthus viridis L., Amaranthus palmeri S. Wats. and Amaranthus ret-roflexus L.; broad leaf weeds such as Solanum carolinense L., Solanum ni-grum L., Chenopodium album L., Abutilon theophrasti medicus, Sida spinosa L., Sesbania exaltata Cory , Ambrosia elatior L., Papaver rhoeas, Ipomoea spp., Xanthium strumarium L., Stel-laria media Villars, Matricaria chamomilla L, Galium spurium L. var. echinospermon Hayek, Viola lanceolata L., Veronica persica Poir., Veronica hederaeforia L., Lalium amplexicaule L., Vicia angustifolia L., Senecio vulgaris L. and Capsella Bursa-pastoris (L.) medik; perennial or anuual sedge cyperaceous weeds such as Cyperus rotundus L., Cyperus esculentus L., Cy-perus brevifolius Hassk. var. leiolepis T. Koyama, Cyperus microiria Steud. and Cyperus iria L.; poaceous weeds such as Echinochloa crus-galli (L.) Beauv. var. paraticola Ohwi, Digitaria ciliaris (Retz.) Koel., Setaria viridis (L.) P. Beauv., Poa annua L., Alopecurus aequalis Sobol. var. amurensis Ohwi, Sorghum halepense Pers., Alopecurus myosuroides Huds., Lolium multiflorum Lamarck. and Avena fatua L.

Also, the present herbicide can control annual weeds, such as Echinochloa oryzicola Vasing, Echinochloa crus-galli (L.) P. Beauv. var. crus-galli, Cyperus difformis L., Leptochloa chinensis (L.) Nees, Monochoria vaginalis Presl var. plan-taginea (Roxb.) Solms-Lau., Lindernia dubia (L.) Pennell, Lin-dernia procumbens (Krock.) Philcox., Rotala indica (Willd.) Koehne var. uliginosa (Miq.) Koehne, Lindernia angustifolia (Benth.) Wettst., Limnophyla sessiliflora Blume, Ammannia multiflora Roxb., Elatine triandra Schk., Monochoria korsakowii Regel et Maack, Ludwigia epilobioides Maxim., Eclipta prostrata L., Bidens Fron-dosa L., Aeschynomene indica L. and Murdannia keisak Hand-Mazz., and perennial weeds such as Sagittaria pygmaea Miq., Sagittaria tri-folia L., Cyperus serotinus Rottb., Eleocharis kuroguwai Ohwi, Scirpus juncoides Roxb., Alisma canaliculatum A. Br. et Bouche, Scirpus nipponicus Makino, Scirpus plani-culmis FR. Schmidt, Potamogeton distinctus A. Bennet, Leersia japonica Makino, Paspalum distichum L., Leersia oryzoides (L.) Swartz and Eleocharis acicularis Roem. et Schult. var. longiseta Svenson, emerging in paddy field.

The herbicide of the present invention shows high safety to useful plants and useful crops and is highly safe, for example, to cereals such as rice, wheat, barley, oat, rye, Italian millet, millet, corn, grain sorghum and the like; soybean; cotton; sugar beat; sugar cane; onion; sunflower; rape; peanut; flax; tobacco; coffee; sweet potato; potato; tomato; other vegetables; Japanese lawngrass.

The useful plants and useful crops include genetically modified crops which have been transformed by genetic engineering and are resistant to herbicides, injurious insects, disease damages, etc., such as corn, soybean, cotton, rape, sugar cane, etc.; and plants which are resistant to herbicides, injurious insects, disease damages, etc. as a result of breeding and selection.

### EXAMPLES

The compound of the present invention, i.e. an optically active (R)-2'-(4,5-dimethoxypyrimidin-2-yl)hydroxylmethyl-1,1-difluoromethanesulfonanilide derivative can be produced by a method shown in the following Production Example.

### <Production Example>

### Production of (R)-2'-(4,6-dimethoxypyrimidin-2-yl)hydroxymethyl-6'-methoxymethyl-1,1-difluoromethanesulfonanilide (compound No. 1)

2.5 g of a racemate, i.e. (RS)-2'-(4,6-dimethoxypyrimidin-2-yl)hydroxymethyl-6'-methoxymethyl-1,1-difluoromethanesulfonanilide (compound No. 2) was dissolved in 25 ml of acetone. The solvent was distilled off under reduced pressure. Right before a crystal separated out, 250 ml of a mixed solvent (n-hexane/2-propanol/acetic acid = 50/50/1) was added.

High-performance liquid chromatography analysis was made using an ultraviolet absorption detector (240nm) under the condition of a flow rate of 5.0 ml/min and room temperature, using, as a column, CHIRAL PAK AD (produced and marketed by Daicel Chemical Industries, Ltd.) (inner diameter 2 cm x length 25 cm) and also using, as a mobile phase, a mixed solvent (n-hexane/2-propanol/aaetic: acid = 50/50/1), whereby a peak of 20 minutes retention time (peak 1) and peak of 30 minutes retention time (peak 2) were obtained.

Incidentally, there was filled, in the column, a silica gel on which a resolving agent, i.e. 1,4-polo-2,3,6-tri-o-phenylcarbamoyl-β-D-glucoside was loaded.

Each peak component was separately obtained repeatedly. The separated solution was concentrated at 60°C under reduced pressure, whereby were obtained 1.07 g of a crystal corresponding to the peak 1 and 1.03 g of a crystal corresponding to the peak 2. Each crystal was recrystallized with isopropyl alcohol for purification to obtain 0.36 g of a crystal corresponding to the peak 1 and 0.32 g of a crystal corresponding to the peak 2. Respective optical rotations were measured. As a result, the peak 1 component showed a specific rotary power of [α]_{D}²⁷ = +245° and the peak 2 component showed a specific rotary power of [α] _{D}²⁷ = -245° (C = 1.00/chloroform).

An X-ray analysis for crystal structure revealed that the peak 2 compound was intended (R)-2'-(4,6-dimethoxypyrimidin-2-yl) hydroxymethyl-6'-methoxymethyl-1,1-difluoromethanesulfonanilide (compound No. 1) and the peak 1 compound was intended (S)-2'-(4,6-dimethoxypyrimidin-2-yl)hydroxyl-methyl-6'-methoxymethyl-1,1-difluoromethanesulfonanilide (compound No. 3).

Next there are specifically described the methods for making the herbicide of the present invention into representative formulations.

Incidentally, the kinds of compound and additives and their compounding ratios are not restricted to the following cases alone and can be changed in a wide range. Incidentally, in the following description, "parts" means mass parts.

### <Formulation Example 1> Wettable powder

With 10 parts of a present invention compound (compound No. 1) were mixed 0.5 part of polyoxyethylene octylphenyl ether, 0.5 part of sodium salt of β-naphthalenesulfonic acid formalin condensate, 20 parts of diatomaceous earth and 69 parts of clay. They were ground to obtain a wettable powder.

### <formulation Example 2> Flowable

20 parts of a coarsely ground present compound (compound No. 1) were dispersed in 69 parts of water. Thereto were added 4 parts of polyoxyethylene styrenated phenyl ether sulfuric acid salt, 7 parts of ethylene glycol and 200 ppm (based on the total volume) of Silicone AF-118N (a product of Asahi Chemical Industry Co., Ltd.). They were mixed for 30 minutes using a high-speed mixer, followed by grinding using a wet grinder, to obtain a flowable agent.

### <Formulation Example 3> Emulsifiable concentrate

To 30 parts of a present compound (compound No. 1) were added 60 parts of an equal-volume mixture of xylene and isophorone and 10 parts of a mixture of surfactants (polyoxyethylene sorbitan alkylate, polyoxyethylene alkyl aryl polymer and alkyl aryl sulfonate). They were stirred to obtain an emulsifiable concentrate.

### <Formulation Example 4> Granule

10 parts of water was added to 10 parts of a present compound (compound No. 1), 80 parts of a mixed solid carrier (talc:bentonite = 1:3), 5 parts of white carbon, 5 parts of a mixture of surfactants (polyoxyethylene sorbitan alkylate, polyoxyethylene alkyl aryl polymer and alkyl aryl sulfonate). They were kneaded sufficiently to obtain a paste. The paste was extruded through sieve openings each of 0.7 mm in diameter, followed by drying and cutting into a length of 0.5 to 1 mm, to obtain a granule.

### <Test Example>

A paddy field soil was filled in a plastic pot having an area of 1/5000 are. Beginning of irrigation, fertilizer application and puddling were conducted. Then, seeds of Echinochloa oryzicola Vasing, Monochoria vaginalis Presl var. plan-taginea (Roxb.) Solms-Lau. and Scirpus juncoides Roxb. were sowed at a depth of 0.5 cm, and submerging was conducted so as to give a water depth of 4 cm. 3 days after the sowing, the wettable powder prepared as in the formulation 1 was diluted with water and dropwise added onto the water surface so that the active ingredient amount (g/10 are) added became the level shown in Table 2. Then, breeding was made at room temperature and, after 30 days, herbicidal effect was examined according to the yardstick shown in Table 1. The results are shown in Table 2.

**Table 1**

| Index | Herbicidal effect (degree of growth control) |
|---|---|
| 10 | Herbicidal effect of 100% control |
| 9 | Herbicidal effect of 90 inclusive to 100% exclusive |
| 8 | Herbicidal effect of 80% inclusive to 90% exclusive |
| 7 | Herbicidal effect of 70% inclusive to 80% exclusive |
| 6 | Herbicidal effect of 60% inclusive to 70% exclusive |
| 5 | Herbicidal effect of 50% inclusive to 60% exclusive |
| 4 | Herbicidal effect of 40% inclusive to 50% exclusive |
| 3 | Herbicidal effect of 30% inclusive to 40% exclusive |
| 2 | Herbicidal effect of 20% inclusive to 30% exclusive |
| 1 | Herbicidal effect of 10% inclusive to 20% exclusive |
| 0 | Herbicidal effect of less than 10% |

**Table 2**

| Compound No. | Dose (g ai/10 a) | Echinochloa oryzicola | Monochoria vaginalis | Scirpus juncoides |
|---|---|---|---|---|
| 1 | 0.32 | 9 | 10 | 10 |
| | 0.16 | 9 | 10 | 10 |
| | 0.08 | 6 | 9 | 9 |
| | 0.04 | 1 | 0 | 7 |
| 2 | 0.32 | 7 | 10 | 10 |
| | 0.16 | 5 | 9 | 9 |
| | 0.08 | 3 | 0 | 8 |
| | 0.04 | 1 | 0 | 2 |
| 3 | 0.32 | 0 | 0 | 0 |

## Claims

1. An optically active (R)-2'-(4,6-dimethoxypyrimidin-2-yl) hydroxymethyl-1,1-difluoromethanesulfonanilide derivative represented by the formula (I) 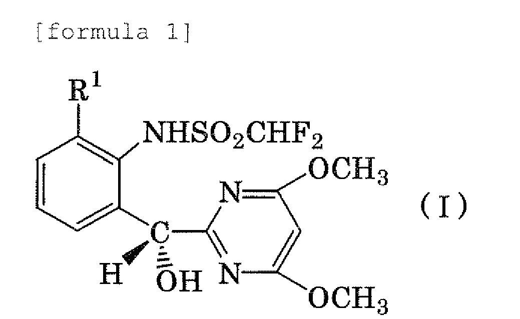 (in the formula, R¹ is a hydrogen atom, a C1-C4 alkyl group or a C1-C4 alkoxy C1-C4 alkyl group).

2. An optically active (R)-2'-(4,6-dimethoxypyrimidin-2-yl)hydroxymethyl-1,1-difluoromethanesulfonanilide derivative according to Claim 1, wherein R¹ is a methoxymethyl group.

3. A herbicide **characterized by** containing, as an active ingredient, an optically active (R)-2'-(4,6-dimethoxypyrimidin-2-yl)hydroxymethyl-1,1-difluoromethanesulfonanilide derivative according to Claim 1 or Claim 2.

4. A method for producing an optically active (R) -2' - (4,6-dimethoxypyrimidin-2-yl)hydroxymethyl-1,1-difluoromethanesulfonanilide derivative, **characterized by** subjecting to optical resolution a racemate of 2'-(4,6-dimethoxypyrimidin-2-yl)hydroxymethyl-1,1-difluoromethanesulfonanilide derivative.
